# EUROPEAN PATENT SPECIFICATION

(11) **EP 1 053 018 B1**
(45) Date of publication and mention of the grant of the patent: **27.07.2005**
(21) Application number: 99902800.4
(22) Date of filing: 29.01.1999
(51) Int. Cl.: A61K 39/39, A61K 39/385, A61K 39/21, A61K 38/19, A61K 39/106, A61K 39/085, A61K 39/00, A61K 39/29

(54) **DELIVERY OF IMMUNOGENIC MOLECULES VIA HBsAg PARTICLES**
LIEFERUNG VON IMMUNOGENEN MOLEKÜLEN UNTER VERWENDUNG VON HBSAG PARTIKELN
APPORT DE MOLECULES IMMUNOGENES VIA DES PARTICULES DE HBsAg

(30) Priority: 03.02.1998 US 73476 P
(43) Date of publication of application: 22.11.2000
(73) Proprietor: YISSUM RESEARCH DEVELOPMENT COMPANY of the Hebrew University of Jerusalem, 91042 Jerusalem (IL); Reimann, Jörg, 89081 Ulm-Lehr (DE)
(72) Inventor: REIMANN, Jorg, D-89081 Ulm-Lehr (DE); BARENHOLZ, Yechezkel, 93707 Jerusalem (IL); DIMINSKY, Dvorah, 96668 Jerusalem (IL); SCHIRMBECK, Reinhold, D-86381 Krumbach (DE)
(74) Representative: Dehmel, Albrecht, Dr.
(86) International application number: PCT/IL1999/000060
(87) International publication number: WO 1999/039736

(56) References cited:
- EP-A- 0 326 109
- EP-A- 0 983 289
- CHEMICAL ABSTRACTS, vol. 129, no. 16, 19 October 1998 (1998-10-19) Columbus, Ohio, US; abstract no. 201834, D. QU ET AL.: "Humoral immune response enhanced by immunization with anti-HBs and HBsAg complex plus DNA vaccine." page 539; column l; XP002109529 & SHANGHAI YIXUE, vol. 20, no. 12, 1997, pages 699-701, China
- H. DAVIS ET AL.: "CpG DNA is a potent enhancer of specific immunity in mice immunized with recombinant hepatitis B surface antigen." THE JOURNAL OF IMMUNOLOGY, vol. 160, no. 2, 15 January 1998 (1998-01-15), pages 870-876, XP002109526 Baltimore, MD, USA
- R. SCHIRMBECK ET AL.: "Nucleic acid vaccination primes hepatitis B virus surface antigen-specific cytotoxic T lymphocytes in nonresponder mice." JOURNAL OF VIROLOGY, vol. 69, no. 10, October 1995 (1995-10), pages 5929-5934, XP002109527 Washington, DC, USA
- R. SCHIRMBECK ET AL.: "Enhancing the immunogenicity of exogenous hepatitis B surface antigen-based vaccines for MHC-I-restricted T cells." BIOLOGICAL CHEMISTRY, vol. 380, no. 3, March 1999 (1999-03), pages 285-291, XP002109528 Berlin, Germany
- Qu et al: (1998) Viral Immunol. 11, 65-72.

## Description

### Field of the Invention

The present invention relates to the use of an antigenic molecule, such as an antigenic peptide, or a cytokine, contained in an HBsAg particle, for the preparation of pharmaceutical compositions, particularly for enhancing the immunogenic activities of the components.

### References

Aspinall, G.O. *et al., Adv. Carbohydr. Chem. Biochem*. **51**:169-242 (1995).
Clarke, B.E. *et al., Nature* **330**(26):381-4 (1987).
Delpeyroux, F. *et al., Science* **233**:472-81 (1986).
Diminsky, D. *et al., Vaccine* **15**(6/7):637-647 (1997).
Felgner, P.L. *et al., Biochemistry* **20**(8):2168-2172 (1981).
Francis, M.J. *et al., Proc. Natl. Acad. Sci. USA* **87**:2545-9 (1990).
Geissler, M. *et al., J. Immunol*. **158**(3):1231-7 (1997).
Lowry, O.H. *et al., J. Biol. Chem*. **193**:265-275 (1951).
Nguyen, T.D. *et al., Cancer. Immunol. Immunother*. **43**(6):345-54 (1997).
Perin, F. *et al., Nucl. Med. Biol.* **21**(8):1093-100 (1994).
Puzo, G., *Crit. Rev. Microbiol*. **17**(4):305-27 (1990).
Schirmbeck, R. *et al., J. Immunol*. **152**(3):1110-1119 (1994).
Talmon, Y., *Ber. Bunsenges. Phys. Chem*. **100**(3):364-72 (1996).
Weiner, G.J. *et al., Proc. Natl. Acad. Sci. USA* **94**(20):10833-7 (1997).
Wong, M. *et al., Biochemistry* **23**:6498-6505 (1984).
Wooldridge, J.E. *et al., Blood* **89**(8):2994-8 (1997).
Yachi, K. *et al., J. Microencapsul*. **12**(4):377-88 (1995).
Yamamoto, S. *et al., J. Immunol*. **148**(12):4072-6 (1992).
Yamamoto, T. *et al., Antisense Res. Dev*. **4**(2):119-22 (1994).

### Background of the Invention

Conventional vaccines against infectious viruses or microorganisms frequently employ inactivated or live-attenuated pathogen. Disadvantages of such vaccine preparations include difficulty in large-scale production, safety considerations in handling, and the risks involved in immunizing elderly or immunodeficient individuals with live-attenuated vaccines.

Subunit vaccines, which utilize isolated components of a virus particle, are a safer alternative to conventional vaccines. The components are typically recombinant proteins or synthetic short peptides. However, most subunit vaccines, like most soluble antigens, generally elicit only a humoral immune response, which stimulates B-lymphocytes to produce antibodies. Such a response is effective in attacking bacteria and viruses in the extracellular media, but not in the elimination of intracellular bacteria, parasites and virus-infected cells. For maximum effectiveness, a vaccine should also be able to elicit a CTL (cytotoxic T-lymphocyte) response. The CTL response stimulates the production of "killer" T-lymphocytes, which attack cells perceived as abnormal, including virus-infected cells.

The mode of processing and presentation of an antigen determines which T cell subtype (helper or cytotoxic) is activated during the immune response. In the exogenous (Class II) pathway, exogenous antigens enter an antigen presenting cell (APC) via endocytosis or a related mechanism. The proteins then undergo proteolysis, yielding peptides having 10-20 amino acids, which bind to MHC-II molecules. The resulting complexes stimulate CD4+ (helper) T cells, which regulate-humoral immune responses. In the endogenous (Class I) pathway, proteins present in the cytoplasm, such as viral proteins, are degraded to peptides 8-10 amino acids in length, which bind to MHC-I molecules. The resulting complexes interact with CD8+ (cytotoxic) T-lymphocytes (CTL). As noted above, this response is especially important for protection against virus-infected cells or intracellular microorganisms.

Accordingly, it is desirable to provide immunogenic compositions which produce an effective CTL immune response, particularly for use with soluble antigens.

### Summary of the Invention

The present invention includes, the use of an antigenic molecule as defined by claim 1. The immune response is a CTL response, and is enhanced, preferably by a factor of two or more, relative to that elicited by the molecules when administered without HBsAg. The compositions are also effective to produce a CTL response when the antigenic molecule, administered without HBsAg, is substantially ineffective in producing such a response.

The HBsAg particle is preferably a recombinant particle, either yeast-derived or produced in a mammalian cell, such as a CHO (Chinese hamster ovary) cell. The encapsulated molecule is preferably an antigenic protein or peptide. Specific embodiments include those in which the molecule is ovalbumin or HIVenv/V3 peptide. In additional embodiments, the composition further includes an immunostimulating molecule, such as a cytokine, contained in the HBsAg particle.

Preferably, the immunostimulating molecule is a cytokine, such as IL-12, IL-10, or IFN-γ; IL-12 and IFN-γ are particularly preferred. Other immunostimulating molecules include cholera toxin (CT) protein, and staphylococcal enterotoxin B (SEB) protein.

The pharmaceutical composition is preferably prepared by incubating the particles in an aqueous medium in the presence of the molecule. In a preferred embodiment, the molecule is an antigen, e.g. HIVenv/K^{d} peptide. In other preferred embodiments, the molecule is an immunostimulating compound, or the particle may contain both an antigen and an immunostimulating molecule.

The composition may also include a glycolipid incorporated into the external face of the lipid bilayer of the HBsAg particle, where the glycolipid preferably includes at least one mannose residue.

In order to incorporate an antigenic molecule into an HBsAg particle, the particles are incubated in an aqueous medium in the presence of the molecule. The temperature of incubation is preferably between about 35°C and about 60°C, and more preferably between about 55°C and about 60°C. The method may also include incorporating a glycolipid into the exterior surface of the HBsAg particle, preferably co-incubating the glycolipid with the HBsAg particles and biologically active molecule.

### Brief Description of the Drawings

Figure 1 shows a computer generated image of a cryotransmission electron micrograph of an HBsAg particle, showing the structure of the porous lipid vesicle having defined protein pores;
Figure 2 is a topographical image of an HBsAg obtained from image analysis of a cryotransmission electron micrograph of the particle;
Figure 3 shows the ratio of total protein area to area of 24kd+27kd proteins, as determined by gel electrophoresis, of HBsAg alone and HBsAg incubated with ovalbumin at a series of increasing temperatures;
Figure 4A shows the level of anti-HBsAg CTL response induced by cells stimulated with HBsAg alone, with OVA alone, and with OVA encapsulated in HBsAg, against HBsAg-specific cells (P815/S) and nonspecific cells (P815);
Figure 4B shows the level of anti-OVA CTL response induced by cells stimulated as for Fig. 4A, against OVA-specific cells (EG7) and nonspecific cells (EL4);
Figure 4C shows the level of anti-HBsAg antibody response induced by HBsAg alone, OVA alone, and OVA encapsulated in HBsAg;
Figure 4D shows the level of anti-OVA antibody response induced by the compositions shown for Fig. 4C;
Figure 5A shows the level of anti-HBsAg CTL response induced by cells stimulated with HBsAg alone, with HIV envV3-peptide alone, and with HIV envV3-peptide encapsulated in HBsAg, against HBsAg-specific cells (P815/S) and nonspecific cells (P815);
Figure 5B shows the level of anti-HIV envV3-peptide CTL response induced by cells stimulated as for Fig. 5A, against HIV envV3-peptide-specific cells (P815/HIV envV3-peptide) and nonspecific cells (P815); and
Figures 6A-H show the level of anti-HBsAg CTL response induced in cells of 'nonresponder' mice (C57BL/6 H2-b) by HBsAg alone (A), HBsAg containing various cytokines (B-F), and IL-12 alone (G), and by HBsAg/IL-12 in a control experiment (H) in which the effector cells were restimulated with non-antigen-bearing cells.

### Detailed Description of the Invention

### I. Hepatitis B Surface Antigen (HBsAg)

### A. Background

Hepatitis B virus (HBV) is a major cause of acute and chronic hepatitis in humans. During HBV replication, a large excess (1000:1) of "empty" surface particles, containing neither capsids nor viral DNA/RNA, are produced. These HBsAg (hepatitis B surface antigen) particles are potent immunogens in humans and many animal species.

First generation HBV subunit vaccines included HBsAg particles purified from the plasma of human chronic carriers. Due to the limited supply of carrier plasma and major safety problems, vaccines based on recombinant HBsAg particles derived from yeast (*Sacchromyces cerevisiae*) were introduced. More recently, a third generation recombinant HBV vaccine, which better resembles the human-derived particles, was introduced. These HBsAg particles are derived from Chinese hamster ovary (CHO) cells in culture and are referred to herein as CHO-HBsAg particles.

### B. Structure

The composition, structure and immunogenicity of yeast- and CHO-derived HBsAg particles have been described (Diminsky *et al*.). The particles are about 20-33 nm in size and are composed of about 60% protein and 40% lipid by weight. Phospholipids are the predominant lipids. The CHO-derived particle differs primarily from the yeast-derived particle in that it includes three HBsAg surface proteins (each in two forms of glycosylation), designated large (L), medium (M) and small (S), while the latter includes only the nonglycosylated S peptide.

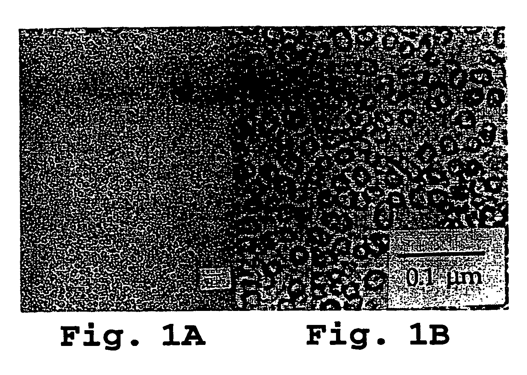

Biochemical analysis revealed that almost all (approx. 85%) HBsAg particle phospholipids are hydrolyzed by phospholipases A2 and C, and all aminophospholipids react with trinitrobenzene sulfonate (Diminsky). These observations suggested that the particle is not a sealed vesicle, but rather exists in the form of (a) a lipoprotein, having a monolayer of polar lipids coating a core of neutral lipids and part of the protein, or (b) a porous vesicle whose pores are permeable to the above reagents. Cryotransmission electron microscopy (Figure 1) confirmed the latter possibility, showing, at a resolution of 1 nm, a porous vesicle.

Phospholipids cover large areas of the outer and inner protein component of the particle, but some protein domains loop out of the lipid layer and are accessible to antibodies or proteases.
The particles are hollow, encapsulating a space of about 900 - 8200 cubic nm per particle. Access to the interior of the particles is mediated by the pores, which have an average diameter of about 1-2 nm.

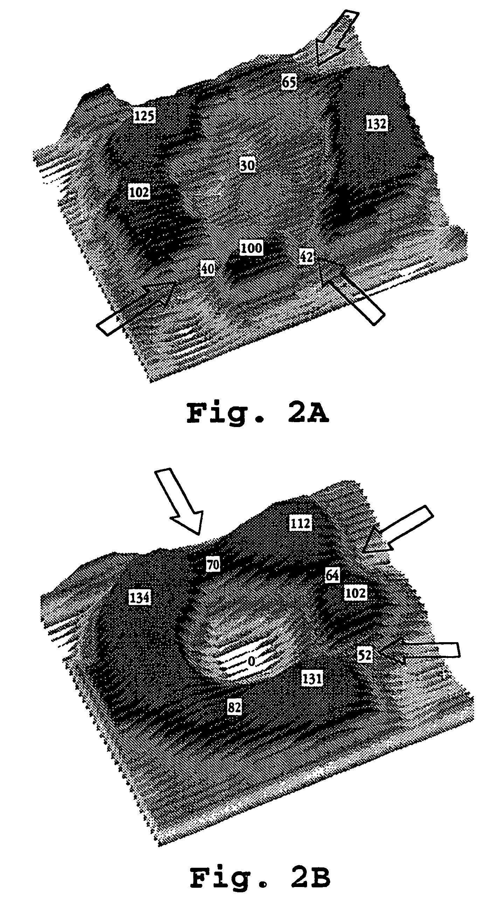

Figures 2A and 2B show topographical images of HBsAg particles, each about 22 nm in diameter, obtained by image analysis of cryotransmission electron micrographs of the particles. (For a review of TEM methods see Talmon, 1996.) Such image analysis can be carried out using software provided by NIH or Adobe Systems Inc. Regions of higher density in the image were assigned higher values along the vertical (out of plane) axis, as shown in Figures 2A-2B. As represented in the Figures, the center of each vesicle contains an aqueous phase (lighter regions, having small numeric values). Proteins (darkest regions, having highest numeric values) are embedded in the lipid bilayer (medium tone regions). Pores in the bilayer can be clearly seen, as indicated by arrows in the Figures.

### II. Encapsulation of Antigens in HBsAg Particles

It has been found that biologically active molecules, such as antigenic proteins and peptides, or cytokines, may be encapsulated in the hollow HBsAg particles, by virtue of the pores described above. As used herein, the terms "encapsulated in" or "contained in" indicate a physical containment or entrapment of the molerules, rather than a covalent linkage, as is found in fusion proteins, discussed further below. This containment refers both to molecule encapsulated within the interior of an HBsAg particle and to entrapped molecule which is exposed or present at the surface of the particle, by virtue of its porous structure.

The resulting compositions produce enhanced immune responses to the encapsulated antigenic molecules. In particular, the compositions can stimulate a CTL response which is enhanced, up to factors of five, ten or more, relative to that elicited by the antigenic molecules when administered without HBsAg. Such enhancement can be evaluated, for example, by percent lysis of specific cells relative to nonspecific control cells, using standard assays, as described below. The compositions can be effective to produce a CTL response even when the molecule, without HBsAg, is substantially ineffective in producing such a response (i.e., little or no CTL response is noted in target cells relative to control cells). As described below in Section III, encapsulation of immunostimulating molecules, such as cytokines, in HBsAg particles greatly enhances the immunogenicity of the HBsAg particles themselves.

These effects are demonstrated, in the experiments described below, for a protein having several hundred amino acids (ovalbumin), a small antigenic peptide (HIV/V3, the third variable domain of the HIV gp120 envelope protein), and several immunostimulants (cytokines).

These examples are not intended to be limiting, and the invention includes the use of HBsAg compositions incorporating other antigenically active molecules. Particularly useful, as demonstrated herein, are compositions incorporating antigens or immunostimulating compounds, for the purpose of eliciting an enhanced humoral and cellular immune response. Specific examples of molecules useful in the compositions include cytokines such as IL-6, IL-7, IL-10, IL-12, IL-15, IL-18, GM-CSF, and IFN-γ, genetically modified toxin molecules of tetanus, diphtheria, pertussis, and enterobacteria, malaria CS protein, HIV reverse transcriptase; nucleoprotein and matrix proteins of many viruses, and oncogenic viral proteins.

It is important to distinguish compositions, which comprise molecules encapsulated in HBsAg particles, from HBsAg-antigen fusion peptides described previously (e.g. Clarke *et al*., Francis *et al*., and Delpeyroux *et al*.). Such compositions are prepared by covalently linking the peptides, or, more typically, via chimeric DNA constructs. The latter approach requires preparing a chimeric DNA containing genes expressing the antigen and the desired HBV protein(s), introducing the fused construct into an appropriate expression vehicle, expressing the fusion protein, and isolating the protein. Delpeyroux *et al*. reported that titers obtained by immunizing mice with HBsAg-polio fusion protein were "low by poliovirus standards." A loss of the immune response against native HB was also observed, probably resulting from distortion of the HBsAg epitopes in the fusion protein. Clarke *et al*. reported significant anti-FMDV (foot and mouth disease virus) titers for an FMDV-HBsAg fusion protein but did not report a CTL response.

The compositions used herein, in contrast, are prepared by simple incubation of the components, not involving covalent modification. They were found to stimulate significant antibody and CTL responses, as detailed below.

### A. Encapsulation of OVA (Ovalbumin) in HBsAg

HBsAg particles were incubated in the presence of OVA protein (100 µg each in 100 µl H₂O) at 4°C (on ice), 37°C and 56°C. At the end of 10 min., samples were cooled to 4°C, and the particles were isolated by ultrafiltration. In control experiments, OVA was incubated under identical conditions in PBS buffer, with no HBsAg particles present, and HBsAg particles were incubated under identical conditions without OVA. The OVA/HBsAg collected after ultrafiltration was analyzed by gel electrophoresis, and the protein in each band was quantified (Diminsky, Lowry). Because OVA overlaps with the PRE S1 (large protein) band of HBsAg, both having a MW of approximately 42kD, analysis was done by comparing total density (39kD + 42kD + 45kD) to that of the two S peptides (24kD + 27kD).

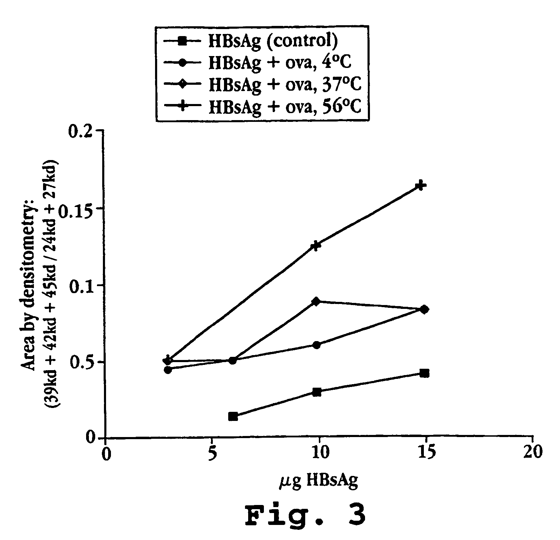

As shown in Fig. 3, the density ratio, and thus the presence of OVA, increased with increasing temperature of incubation. This effect could be due to an effective expansion, or increased flexibility, of the pores on the surface of the particles with increasing temperature. Quantitative analysis revealed about 6% OVA encapsulation at 56°C. Incubation temperatures much in excess of this, e.g. approaching 80°C, should be avoided, as the compositions are unstable at these temperatures.

### B1. Induction of a CTL Response to Molecules Encapsulated in HBsAg Particles: OVA

OVA/HBsAg particles, prepared as described above, were isolated, washed, and adjusted to the appropriate concentration for immunization. The following compositions were injected into F1 (H-2d/b) mice, each in 50 µl PBS (phosphate buffered saline): (a) 1 µg HBsAg particles (without adjuvants); (b) 100 µg native OVA; and (c) 1 µg HBsAg/OVA, as described in Section A above.

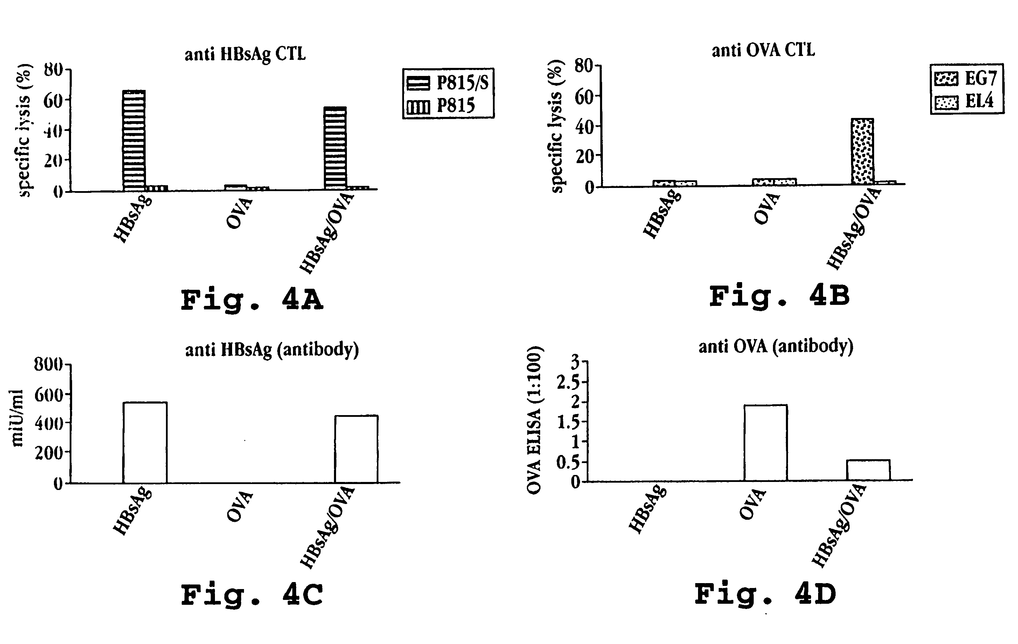

Figures 4A and 4B show the humoral (serum antibody) response and CTL response, respectively, elicited by each of these compositions, against HBsAg and against OVA. For evaluation of the CTL response, spleen or lymph nodes cells, obtained from immunized mice seven days to five weeks post-vaccination, were specifically restimulated *in vitro* for five days with syngeneic, irradiated OVA- or HBsAg-peptide-pulsed tumor cells. (The latter cells had been incubated in vitro with the recombinant HBsAg particles for 2 hours at 37°C.) The cells were harvested and tested in a 4-hour ⁵¹Cr release cytolytic assay against antigen-bearing and non-antigen-bearing syngeneic targets. Lysis of cells specific for the HBsAg S protein (P815/S), or for OVA (EG7), was compared to that for nonspecific cells (P815 or EL4, respectively). The EG7 cells are EL4 cells which have been stably transfected with an expression plasmid encoding OVA.

As shown in the figures, composition (a), HBsAg alone, evoked both a CTL response and a humoral response against HBsAg. OVA alone elicited a humoral response but no CTL response.

HBsAg-encapsulated OVA, composition (c), elicited a strong anti-HBsAg humoral and CTL response and a weak anti-OVA humoral response. Most significantly, the composition also elicited a strong anti-OVA CTL response, where none was seen with OVA alone.

### B2. Induction of CTL Response to HBsAg-Encapsulated Molecules: HIVenv/V3 Peptide

In a similar set of experiments, BALB/c (H-2d) mice were immunized with the following compositions: (a) 1 µg HBsAg particles (without adjuvants); (b) 100 µg antigenic HIVenv/V3 peptide; and (c) 1 µg HBsAg containing antigenic HIVenv/V3 peptide. This composition was formed by co-incubation of the components, generally as described for OVA, above. It was estimated that approximately 20 ng peptide was incorporated per µg of HBsAg particles.

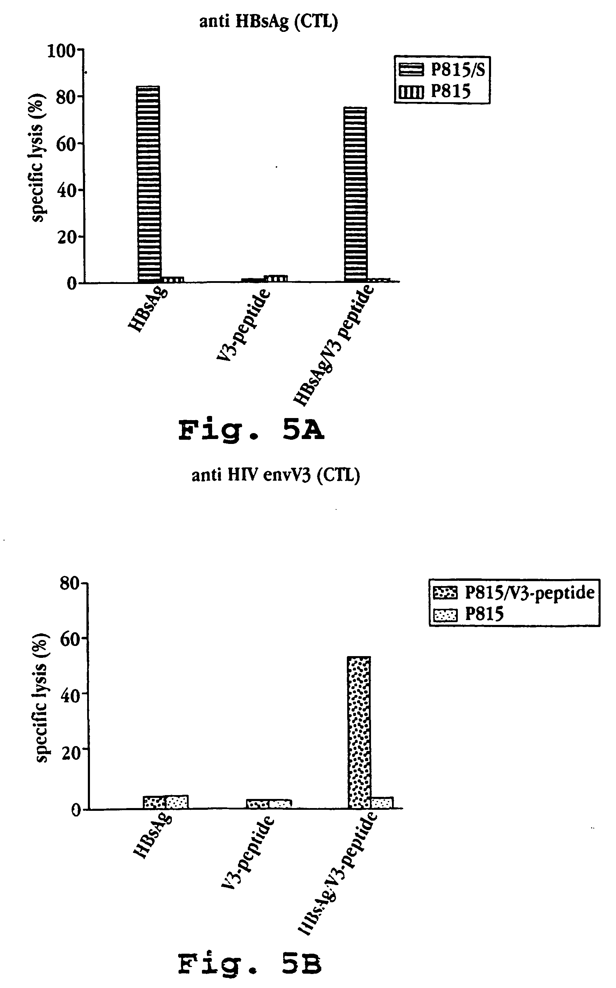

The CTL response was measured in specific cells (P815/S, as described above, for HBsAg, and P815/V3-peptide for HIVenv/V3 peptide) vs. that in nonspecific P815 cells. The results are shown in Figure 5.

As shown in the Figure, both HBsAg alone and HBsAg/HIVenv/V3 elicited a strong anti-HBsAg CTL response. Furthermore, while HIVenv/V3 peptide alone (composition (b)) elicited no specific CTL response, a strong anti-HIVenv/V3 CTL response was seen for the peptide delivered in HBsAg (composition (c)).

These results show that proteins and peptides can be delivered to antigen-presenting cells (APC) *in vivo* by HBsAg particles for processing and immunogenic presentation via the Class I pathway, thus stimulating CTL precursors. This CTL response is elicited even for antigens that are not immunogenic for CTL when injected as native proteins.

In a related embodiment of this method, codelivery of antigen and cytokine in HBsAg can be employed to modulate the type of immune response primed or enhanced by a HBsAg/antigen formulation. For example, IL-12 drives CD4+ T-cell response polarization towards the Th1 phenotype and suppresses the Th2 phenotype. In contrast, IL-10 suppresses Th1-type response and enhances Th2-type response. Such compositions are useful when it is desired to modulate the pathogenic phenotype of an autoimmune or allergic T-cell response. For example, in treatment of autoimmune disease, it is often desirable to shift the phenotype of the immune response, rather than suppressing the response entirely.

### III. Modulation of CTL Response by Immunostimulants Encapsulated in HBsAg Particles

HBsAg particles, without adjuvants, induce a CTL response in H-2^{d}/L^{d+} (BALB/c, C.B-17) mice. Other strains of mice, e.g. H-2^{d}/L^{d-} (dm2) and H-2^{b} (C57BL/6) mice, however, were found to be nonresponders (Schirmbeck *et al*.). It has been found, that encapsulation of immunostimulating molecules, e.g. cytokines, in HBsAg particles can induce a CTL response even in these 'nonresponder' strains.

HBsAg particles can also be used to deliver immunostimulatory oligonucleotides. Such a composition enhances the immunogenicity of the HBsAg for B cells (i.e., the antibody response) as well as T cells (CTL response). For example, oligonucleotides containing certain palindromic sequences were found to induce IFN and augment NK cell activity of mouse spleen cells (Yamamoto *et al*., 1992, 1994). Other oligonucleotides have been effective as adjuvants in inducing production of cytokines, activating B cells, monocytes, dendritic cells, and NK cells (Weiner; Woolridge).

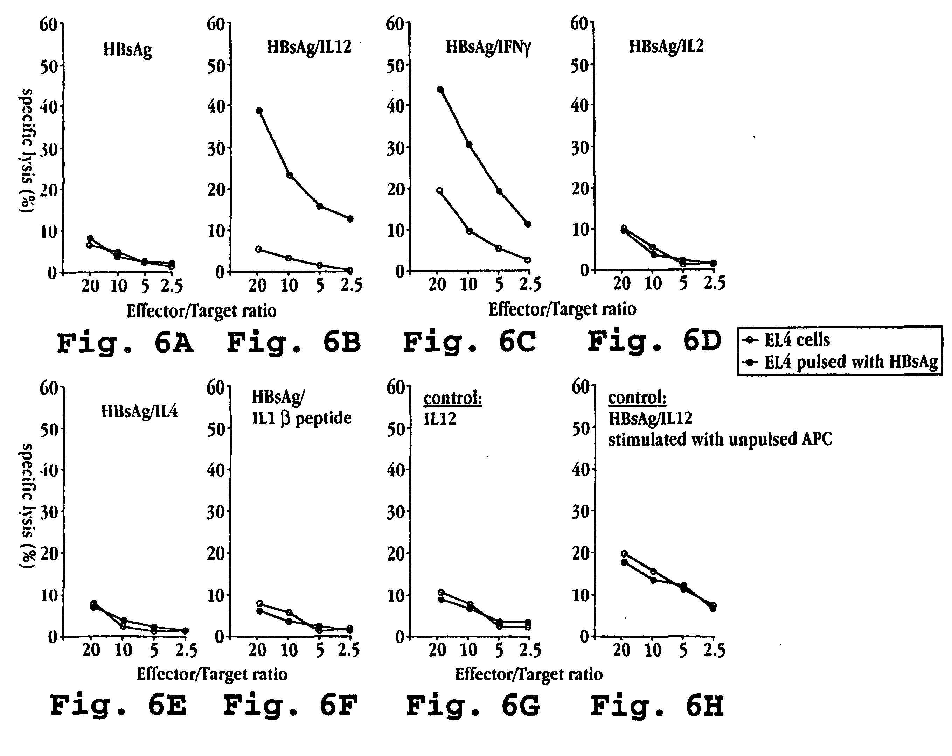

To produce the data shown in Figure 6, several cytokines were loaded into HBsAg particles, following a protocol such as outlined above for OVA, with incubation carried out at 45°C. It was estimated that approximately 10 ng of cytokine was incorporated per µg of HBsAg particles (about 1% incorporation) at this temperature. Groups of 'nonresponder' mice (H-2^{b} C57BL/6) were immunized with HBsAg particles, with and without incorporated cytokines, or with cytokine alone (Fig. 6G), in the amounts shown below:
A: 1 µg 'naked' HBsAg particles
B-F: 1 µg HBsAg/cytokine, where the cytokine was:
   (b) IL-12, (c) IFN-γ, (d) IL-2, (e) IL-4, (f) IL-1 β peptide
G: 1 µg IL-12 (control)
H: 1 µg HBsAg/IL-12

After 12 days, splenocytes from the immunized mice were restimulated in vitro for 5 days with HBsAg-pulsed, syngeneic irradiated RBL5 lymphoma cells. For control experiment H, the splenocytes were restimulated with non-pulsed, syngeneic RBL5 cells. The splenocytes (effector cells) were then cocultured with ⁵¹Cr-labeled target cells, and the CTL response was measured by a standard ⁵¹Cr release assay. The target cells were either non-pulsed EL4 cells (open circles in Figs. 6A-G), or EL4 cells which had been pulsed with HBsAg (solid circles).

Results are shown in Figs. 6A-H. In the control experiments (A and G), where the "nonresponder" mice were immunized with HBsAg alone or IL-12 alone, no CTL response was seen in the HBsAg-specific cells as compared to the control cells. Nor was any response seen in experiment H, in which the splenocytes were restimulated with non-antigen-bearing RBL5 cells.

Compositions D-F showed a similar lack of response. The lack of response from HBsAg encapsulating IL-4 (E) is not unexpected, as this cytokine is known to suppress the CTL response (see, for example, Nguyen, Geissler).

A strong CTL response was seen, however, for compositions B and C, where the cytokines were IL-12 and IFN-γ, respectively. This data shows that encapsulation of certain cytokines in HBsAg particles can induce a CTL response even in "nonresponder" strains; i.e., in subjects which do not show a CTL response to HBsAg alone.

In further experiments, the immunostimulating proteins cholera toxin (CT) and staphylococcal enterotoxin B (SEB), known as adjuvants for stimulation of CTL and humoral immune responses, were each incorporated into HBsAg particles, at a level of about 5-20 ng protein per µg HBsAg. Preliminary experiments testing the immunogenicity of these compositions showed a striking enhancement of the CTL and antibody responses of the HBsAg particles.

### IV. Incorporation of Glycolipids into the Outer Surface of HBsAg Particles

Glycolipids can be introduced into the HBsAg particle exterior membrane by lipid exchange, using either micelles or liposomes, as described in Felgner. In Felgner, co-incubation of ganglioside micelles (containing predominantly trisialoganglioside GT1b) with fused phosphatidyl choline vesicles, about 70 nm in diameter, or SUV, about 20 nm in diameter, resulted in incorporation of ganglioside on the outer surface of the vesicles. Alternatively, the glycolipids can be introduced by the use of a glycolipid exchange protein, as described in Wong *et al*.

By such inclusion of glycolipids, particularly glycolipids containing available mannose residues, the particles may be targeted to specific antigen presenting cells, such as dendritic cells or macrophages. See, for example, Yachi, where modification of the surface of liposomes with fatty acid esters of mannobiose was found useful in targeting the liposomes to Kupffer cells and other macrophages. Perin *et al*. have employed an acylated poly-(1,3)-galactoside for the targeting of macrophages.

The surface glycolipids of mycobacteria have been well characterized (see e.g. Aspinall, Puzo). These glycolipids, which are often species-specific, have been used for the identification of various species, such as *M. leprae* and *M. tuberculosis*, and for monitoring treatment of disease caused by these organisms. In accordance with the present invention. HBsAg having a specific glycolipid incorporated into the lipid monolayer may be administered to induce a CTL response against mycobacteria-infected, syngeneic cells.

### V. Administration

For use in humans, a preferred dose of encapsulated antigen is in the range of 0.01 to 20 µg, more preferably 0.02 to 2 µg, incorporated at about a 1 to 10 weight percent level in HBsAg particles. Administration may be by injection, e.g., intraperitoneal (ip), subcutaneous (sc), intravenous (iv), or intramuscular (im).

While the invention has been described with reference to specific methods and embodiments, it will be appreciated that various modifications may be made without departing from the invention as defined by the appended claims.

## Claims

1. Use of an antigenic molecule encapsulated in an Hepatitis B surface Antigen (HBsAg) particle for the preparation of a pharmaceutical composition for stimulating or modulating a CTL response to the antigenic molecule, wherein said antigenic molecule is not covalently attached to said HBsAg particle, and the antigenic molecule is an antigenic peptide or protein.

2. Use of claim 1, wherein said CTL response is enhanced relative to that produced by the antigenic molecule alone.

3. Use of claim 1 or 2, wherein said pharmaceutical composition further comprises an immunostimulating molecule encapsulated in said HBsAg particle.

4. Use of claim 3, wherein said immunostimulating molecule is a cytokine.

5. Use of claim 1, wherein said antigenic protein or peptide is ovalbumin or the HIVenv/V3 peptide.

6. Use of claim 1, wherein said immunostimulating molecule is cholera toxin (CT) protein or staphylococcal enterotoxin B (SEB) protein.

7. Use of any one of claims 1 to 6, further comprising a glycolipid incorporated into the exterior surface of the lipid bilayer of said HBsAg particle.

## Patentansprüche

1. Verwendung eines in einem Hepatitis B-Oberflächenantigen (HBsAg)-Partikel verkapselten antigenen Moleküls für die Zubereitung einer pharmazeutischen Zusammensetzung zur Stimulation oder Modulation einer CTL-Reaktion auf das antigene Molekül, wobei das antigene Molekül an dem HBsAg-Partikel nicht kovalent befestigt ist und das antigene Molekül ein antigenes Peptid oder Protein ist.

2. Verwendung gemäß Anspruch 1, wobei die CTL-Reaktion im Vergleich zu der, die durch das antigene Molekül allein hervorgerufen wird, verstärkt ist.

3. Verwendung gemäß Anspruch 1 oder 2, wobei die pharmazeutische Zusammensetzung weiter ein immunstimulierendes Molekül umfasst, das in dem HBsAg-Partikel verkapselt ist.

4. Verwendung gemäß Anspruch 3, wobei das immunstimulierende Molekül ein Zytokin ist.

5. Verwendung gemäß Anspruch 1, wobei das antigene Protein oder Peptid Ovalbumin oder das env/V3-Peptid von HIV ist.

6. Verwendung gemäß Anspruch 1, wobei das immunstimulierende Molekül das Choleratoxin (CT)-Protein oder Staphylokokken Enterotoxin B (SEB)-Protein ist.

7. Verwendung gemäß einem der Ansprüche 1 bis 6, weiter umfassend ein Glykolipid, das in die äußere Oberfläche der Lipid-Doppelschicht des HBsAg-Partikels eingebettet ist.

## Revendications

1. Utilisation d'une molécule antigénique encapsulée dans une particule d'Antigène de surface de l'Hépatite B (HBsAg) pour la préparation d'une composition pharmaceutique pour stimuler ou moduler une réponse CTL contre la molécule antigénique, dans laquelle ladite molécule antigénique n'est pas attachée de manière covalente à ladite particule HBsAg, et la molécule antigénique est un peptide ou une protéine antigénique.

2. Utilisation selon la revendication 1, dans laquelle ladite réponse CTL est augmentée par rapport à celle produite par la molécule antigénique seule.

3. Utilisation selon la revendication 1 ou 2, dans laquelle ladite composition pharmaceutique comprend en outre une molécule immunostimulante encapsulée dans ladite particule HBsAg.

4. Utilisation selon la revendication 3, dans laquelle ladite molécule immunostimulante est une cytokine.

5. Utilisation selon la revendication 1, dans laquelle ladite protéine ou ledit peptide antigénique est l'ovalbumine ou le peptide HIVenv/V3.

6. Utilisation selon la revendication 1, dans laquelle ladite molécule immunostimulante est la toxine du choléra (CT) ou l'entérotoxine B staphylococcique.

7. Utilisation selon l'une quelconque des revendications 1 à 6, comprenant en outre un glycolipide incorporé dans la surface extérieure de la bicouche lipidique de ladite particule HBsAg.
